Europäisches Patentamt

European Patent Office

Office européen des brevets

(19)

(11) Numéro de publication: **0 084 755
B1**

(12) **FASCICULE DE BREVET EUROPEEN**

(45) Date de publication du fascicule du brevet:
**19.02.86**

(21) Numéro de dépôt: **82440043.6**

(22) Date de dépôt: **09.12.82**

(51) Int. Cl.⁴: **A 61 F 5/46,** A 61 B 17/42

(54) **Pessaire de grossesse.**

(30) Priorité: **28.01.82 FR 8201526**

(43) Date de publication de la demande:
**03.08.83 Bulletin 83/31**

(45) Mention de la délivrance du brevet:
**19.02.86 Bulletin 86/8**

(84) Etats contractants désignés:
**AT BE CH DE GB IT LI LU NL SE**

(56) Documents cités:
**DE - C - 126 095
FR - A - 2 409 044
US - A - 1 750 188
US - A - 1 790 801**

(73) Titulaire: **SOCIETE ALSACIENNE D'INSTALLATIONS TECHNIQUES, 10 rue du Zornhoff, F-67700 Saverne (FR)**

(72) Inventeur: **Jouffroy, Christian, 24, rue des Carolingiens, F-67000 Strasbourg (FR)**
Inventeur: **Renaud, Robert, 9, quai Lezay Marnésia, F-67000 Strasbourg (FR)**

(74) Mandataire: **Kedinger, Jean-Paul et al, CABINET MALEMONT 13 rue du Général de Castelnau, F-67000 Strasbourg (FR)**

ACTORUM AG

## Description

La présente invention concerne un nouveau pessaire utilisable en particulier pour le traitement des insuffisances cervico-isthmiques lors de la grossesse, constitué par une plaque de forme sensiblement trapézoïdale, délimitée par deux côtés latéraux, un côté antérieur concave et un côté postérieur également concave, mais plus long que le côté antérieur et pourvue d'une part, d'un orifice circulaire destiné à recevoir le col de l'utérus et d'autre part, de perçages pour l'écoulement des sécrétions vaginales.

On rappellera que l'insuffisance ou la béance cervico-isthmique est une incapacité du col et de l'isthme à jouer leur rôle physiologique d'occlusion de l'utérus, indispensable à la protection et au maintien de l'oeuf dans la cavité utérine.

On peut différencier:

— les insuffisances cervico-isthmiques organiques (congénitales ou acquises), dont le diagnostic peut être porté en dehors de la grossesse,

— des insuffisances cervico-isthmiques fonctionnelles dont le diagnostic n'est très généralement porté que pendant la grossesse et qui correspondent à des modifications du col (raccourcissement voire effacement, dilatation) constatées au cours de l'évolution d'une grossesse chez des femmes sans antécédents gynécologiques ou obstréticaux évocateurs.

Ces insuffisances sont responsables pendant la grossesse, des accouchements prématurés et des avortements tardifs. En effet, du fait de l'abaissement de la résistance mécanique du col, les contractions utérines et le poids du foetus entraînent d'autant plus facilement une ouverture du col, éventuellement accompagné d'une hernie des membranes et leurs rupture éventuelle pouvant aboutir à un accouchement prématuré ou à un avortement. Un autre facteur qui semble devoir être pris en considération pour expliquer ces accouchements prématurés et ces avortements tardifs est l'infection des membranes que permet la béance cervico-isthmique par empêchement de la fonction du bouchon muqueux dans l'endocol.

On a donc cherché à mettre au point un moyen permettant de créer, pendant la durée de la grossesse, un nouveau verrou isthmique, c'est-à-dire un moyen apte à renforcer la résistance mécanique du col et à permettre au bouchon muqueux de jouer à nouveau son rôle de barrière contre l'infection et ce, afin que la grossesse puisse être menée à terme.

Un tel moyen peut être constitué par le pessaire et notamment par le pessaire cubique de JORDE. Ce pessaire particulier, qui répond à la définition apparaissant au début de la présente description et dont les côtés latéraux sont concaves et les perçages de faibles dimensions, est cependant relativement lourd, gêne l'écoulement des sécrétions vaginales et surtout ne soutient pas suffisamment le segment inférieur de l'utérus (partie basse antérieure) qui est le siège essentiel de la pression due au poids de l'oeuf ou du foetus. Or, de par la structure même du pessaire cubique, il n'est pas possible d'augmenter la surface destinée à soutenir le segment inférieur, c'est-à-dire la surface située entre l'orifice circulaire et le côté antérieur, sans augmenter simultanément de manière importante, d'une part la taille dudit pessaire qui devient alors incompatible avec l'anatomie de la femme et d'autre part son poids qui met fortement en cause le bon maintien en place.

La présente invention a donc pour but d'améliorer les performances des pessaires existants et pour ce faire, elle propose un pessaire du type de celui décrit au premier paragraphe de la présente description et qui se caractérise en ce que les deux côtés latéraux sont convexes.

Grâce à cette nouvelle conformation, la partie antérieure du pessaire selon l'invention, c'est-à-dire celle située en avant de l'orifice circulaire, est apte à assurer un soutènement accru et très efficace du segment inférieur. De ce fait, la pression créée par l'oeuf ou le foetus peut être uniformément répartie sur les structures ligamentaires et musculo-aponévrotiques situées à la périphérie dudit segment inférieur, l'orifice interne de l'utérus n'étant ainsi soumis qu'à une pression minimale ce qui réduit considérablement les possibilités d'ouverture du col.

Pour des questions de stabilité de l'ensemble, on préfère bien entendu que le centre de l'orifice circulaire soit situé sensiblement sur l'axe antéro-postérieur.

Par ailleurs, on sait que l'insuffisance cervico-isthmique pendant la grossesse se caractérise par une centralisation du col dont l'axe ne forme plus un angle aigu avec l'axe du vagin, mais devient parallèle à ce dernier axe. Dans le but de reconstituer cet angle aigu, le centre de l'orifice circulaire de pessaire selon l'invention sera avantageusement légèrement plus proche du côté postérieur que du côté antérieur.

Selon une autre caractéristique de l'invention, l'orifice circulaire destiné à recevoir le col est délimité par un anneau relié par une branche au moins, à une armature périphérique, cette dernière et la ou les branches de liaison définissant ainsi avec le bord périphérique de l'anneau, les perçages ou ouvertures dont le rôle est d'assurer l'écoulement des sécrétions vaginales. Il en résulte un pessaire fait non pas d'une seule masse comme les pessaires connus, mais d'une structure aérée et légère mais néanmoins rigide.

En choisissant judicieusement la position, le nombre et la forme des branches de liaison, il est possible de réaliser un pessaire qui, tout en assurant un soutènement optimal du segment inférieur, est pourvu de perçages dont la surface totale peut atteindre une valeur suffisamment importante pour permettre un écoulement aisé desdites sécrétions vaginales.

Ainsi, l'une des branches de liaison est avantageusement situé entre l'anneau et le côté antérieur de la plaque et mieux encore, les bords de cette branche divergent de l'anneau vers le côté antérieur.

Selon un mode de réalisation préféré de l'invention, les bords de cette même branche divergent jusqu'à rejoindre respectivement l'armature périphérique au niveau de la partie antérieure des côtés latéraux de la plaque. De cette manière, on définit une plaquette de soutènement plane assurant une parfaite répartition de la pression sur les structures fermes et résistantes situées à la périphérie du segment inférieur. En outre, cette plaquette de soutènement en coopération avec l'anneau, fait office de fronde en bandant le segment inférieur et la lèvre antérieure du col, empêchant ainsi efficacement la descente de la tête du foetus et sa pression sur l'orifice interne.

Selon une autre caractéristique encore de l'invention, le pessaire peut comporter, outre la branche dont la position vient d'être définie, deux autres branches disposées sensiblement symétriquement de part et d'autre de l'axe antéro-postérieur et reliant respectivement l'anneau à l'armature périphérique au niveau des extrémités du côté postérieur.

Un mode de réalisation de la présente invention est décrit ci-après en regard du dessin annexé dans lequel:

la fig. 1 est une vue en plan du pessaire,

la fig. 2 est une coupe effectuée selon la ligne II-II du pessaire représenté par la fig. 1, et

la fig. 3 est une coupe antéro-postérieure partielle du bassin montrant la position du pessaire asprès sa mise en place.

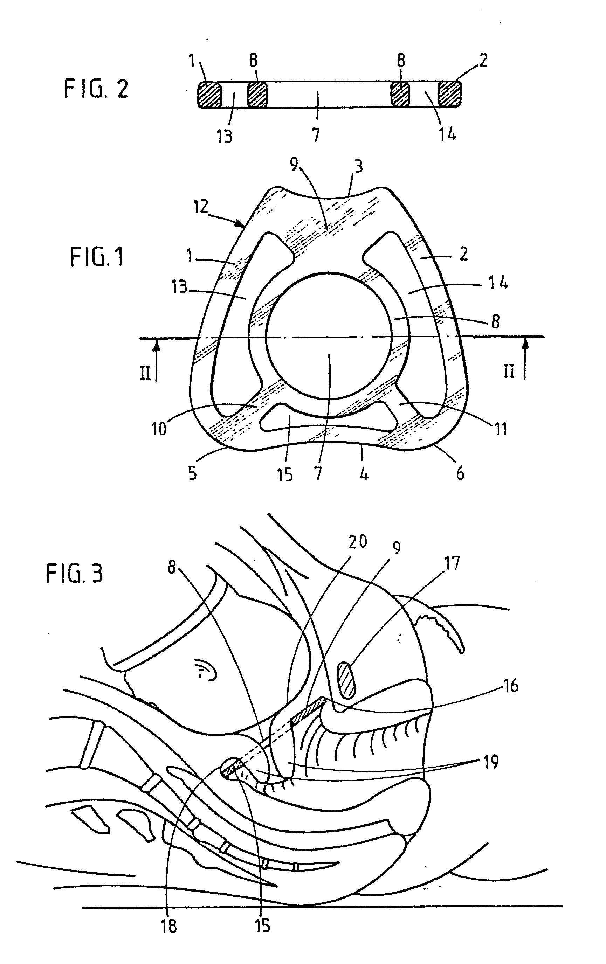

Le pessaire représenté par les figures 1 et 2 est constitué par une plaque rigide ayant sensiblement la forme d'un trapèze isocèle dont les deux côtés latéraux 1, 2 sont de forme convexe, et dont la petite base 3, que nous qualifierons ci-après d'antérieure et la grande base 4 que nous qualifierons ci-après de postérieure sont de forme concave, la base postérieure 4 rejoignant les côtés latéraux 1, 2 par des parties fortement arrondies 5, 6. Cette plaque est par ailleurs pourvue d'un orifice circulaire 7 dont le centre est situé sur l'axe antéro-postérieur de la dite plaque et à une distance de la base postérieure 4 légèrement plus courte que celle qui le sépare de la base antérieure 3.

Plus précisement, l'orifice circulaire 7 est délimité par un anneau 8 relié par trois branches de liaison 9, 10, 11 à une armature périphérique rigide 12 dont la forme extérieure est définie par celle des côtés latéraux 1, 2 et des bases antérieure et postérieure 3, 4. Les bords de la branche 9, située entre l'anneau 8 et la base antérieure 3 et dont l'axe longitudinal est de préférence confondu avec l'axe antéro-postérieur, divergent symétriquement par rapport audit axe antéro-postérieur, dudit anneau 8 vers ladite base antérieure 3, jusqu'à rejoindre l'armature périphérique 12 au niveau de l'extrémité antérieure des côtés latéraux 1, 2. D'autre part, les branches 10, 11, disposées symétriquement de part et d'autre de l'axe antéro-postérieur, partent de l'anneau 8 pour rejoindre respectivement les parties fortement arrondies 5, 6 de la plaque. Ainsi, les branches 9 et 10 d'une part, et les branches 9 et 11 d'autre part, définissent respectivement avec la surface périphérique de l'anneau 8 et les côtés latéraux de l'armature périphérique 12, deux longues ouvertures latérales 13, 14 sensiblement en forme de croissant. De la même manière, les branches 10 et 11 et la surface périphérique de l'anneau 8 définissent avec la base postérieure de l'armature périphérique 12 une ouverture allongée 15.

La corbure des côtés latéraux 1, 2 sera choisie de manière à ce que la partie antérieure du pessaire constitue la plus grande surface possible de soutènement du segment inférieur de l'utérus, sans pour autant gêner la pose dudit pessaire et provoquer l'inconfort de la patiente; cette courbure sera donc en fait essentiellement fonction de la taille et de la morphologie de la patiente, ces deux critères étant également ceux qui conditionnent le choix des dimensions du pessaire. Quant aux courbures des bases antérieure et postérieure 3, 4, elles seront choisies de telle manière que le pessaire une fois mis an place, n'exerce pas une pression exagérée respectivement sur l'urètre et le rectum.

Le diamètre de l'orifice circulaire 7 devra pour sa part être calculé pour assurer le maintien du col et de préférence provoquer un resserrement suffisant de l'orifice interne de ce dernier pour eviter toute dilatation.

Par ailleurs, la position de cet orifice circulaire 7, c'est-à-dire la position de son centre sur l'axe antéro-postérieur sera définie de manière à ramener, si besoin est, l'axe du col dans sa position normale.

Il est bien certain que la position, le nombre et la forme des branches reliant l'anneau 8 à l'armature périphérique 12 pourraient être différents de ce qu'ils sont dans la fig. 1; il apparaît néanmoins que la position, le nombre et la forme présentement illustrés conduisent aux meilleurs résultats cliniques.

L'armature périphérique 12 et l'anneau 8 présentent une section sensiblement rectangulaire, mais cette section pourraît être autre, par exemple carrée ou circulaire, l'essentiel étant que cet anneau et cette armature périphérique ne présentent pas d'arête vive afin d'éviter toute lésion interne; c'est ce que montre la fig. 2 sur laquelle tous les bords ont une forme parfaitement arrondie. Cette remarque vaut d'ailleurs pour toutes les autres parties du pessaire.

L'épaisseur du pessaire dépendra de la densité et de la résistance mécanique du matériau utilisé pour sa réalisation; elle sera choisie de manière à disposer d'un pessaire le plus léger possible mais malgré tout suffisamment rigide pour résister aux pressions auxquelles il sera soumis.

Une dernière remarque s'impose quant au matériau utilisable pour la réalisation du pessaire selon l'invention; il conviendra bien entendu qu'il soit lisse, sans aspérité susceptible de gêner ou de blesser et absolument inerte sur le plan physiologique. Un tel matériau pourra par exemple être constitué par une résine du type Kallokryl A ou polyméthacrylate.

La technique de pose est simple puisqu'elle se pratique à la consultation sans anesthésie. Après lubrification du pessaire par la glycérine, on déprime le périné postérieur, introduit le pessaire de biais, attire le col dans l'orifice circulaire 7 et assure que la base antérieure 3 du pessaire repose bien contre le bord postérieur de la symphyse. Une bonne tolérance immédiate est de règle lorsque le pessair est bien en place et présente des dimensions et courbures adaptées à la morphologie de la patiente. Il est à remarquer que la pose peut en principe être effectuée jusqu'à la 35ème semaine de grossesse et que le retrait doit de préférence être effectué à 37 semaines révolues.

La fig. 3 montre la position du pessaire une fois la pose réalisée. On notera à l'examen de cette figure que la base antérieure 3 du pessaire est logée dans le cul de sac antérieur 16 et repose sur le bord postérieur de la symphyse 17, la base postérieure 4 du pessaire étant pour sa part logée dans le cul de sac postérieur 18. On notera également que le col 19 est maintenu et enserré dans l'anneau 8 dont le rôle est de suppléer au verrou isthmique déficient et que l'ensemble branche de liaison 9 — partie antérieure de l'armature périphérique 12 du pessaire, définit une large surface garantissant un soutènement très efficace du segment inférieur 20 sur lequel s'applique le pois de l'enfant.

Enfin, les ouvertures 13, 14, 15 de par leur surface très importante, permettent l'évacuation totale sinon suffisante des sécrétions vaginales pour eviter une quelconques infection.

## Revendications

1. Pessaire utilisable en particulier pour le traitement des insuffisances cervico-isthmiques lors de la grossesse, constitué par une plaque de forme sensiblement trapézoïdale, délimitée par deux côtés latéraux (1, 2), un côté antérieur concave (3) et un côté postérieur (4) également concave mais plus long que le côté antérieur et pourvue d'une part, d'un orifice circulaire (7) destiné à recevoir le col de l'utérus et d'autre part, de perçages (13, 14, 15) pour l'écoulement des sécrétions vaginales, caractérisé en ce que les deux côtés latéraux sont de forme convexe.

2. Pessaire selon la revendication 1, caractérisé en ce que le centre de l'orifice circulaire (7) est situé sensiblement sur l'axe antéro-postérieur.

3. Pessaire selon la revendication 1 ou 2, caractérisé en ce que le centre de l'orifice circulaire (7) est légèrement plus proche du côté postérieur (4) que du côté antérieur (3).

4. Pessaire selon l'une quelconque des revendications précédentes, caractérisé en ce que l'orifice circulaire (7) est délimité par un anneau (8) relié par une branche au moins (9, 10, 11), à une armature périphérique (12), cette dernière définissant avec la ou les branches de liaison (9, 10, 11) les perçages (13, 14, 15) pour l'écoulement des sécrétions vaginales.

5. Pessaire selon la revendication 4, caractérisé en ce que l'une des branches de liaison (9) est située entre l'anneau (8) et le côté antérieur (3).

6. Pessaire selon le revendication 5, caractérisé en ce que les bords de ladite branche de liaison (9) divergent de l'anneau (8) vers le côté antérieur (3).

7. Pessaire selon la revendication 5 ou 6, caractérisé en ce que les bords de la branche de liaison (9) divergent jusqu'à rejoindre respectivement l'armature périphérique (12) au niveau de la partie antérieur des côtés latéraux (1, 2).

8. Pessaire selon la revendication 5, 6 ou 7, caractérisé en ce qu'il comporte en outre, deux autres branches de liaison (10, 11) disposées sensiblement symétriquement de part et d'autre de l'axe antéropostérieur et reliant respectivement l'anneau (8) à l'armature périphérique (12) au niveau des extrémités du côté postérieur (4).

## Patentansprüche

1. Pessar, das speziell für die Behandlung von durch Schwangerschaft hervorgerufene Insuffizienzen der Gebärmutterhalsverengung verwendbar ist, bestehend aus einer Platte von im wesentlichen trapezförmiger Gestalt, die von zwei Querseiten (1, 2), einer konkaven Vorderseite (3) und einer ebenfalls konkaven, jedoch gegenüber der Vorderseite längeren Hinterseite (4) begrenzt ist und einerseits mit einer kreisförmigen Öffnung (7) zur Aufnahme des Gebärmutterhalses und andererseits mit Durchbrüchen (13, 14, 15) für den Ausfluss von Vaginalsekreten versehen ist, dadurch gekennzeichnet, dass die beiden Querseiten konvexe Gestalt haben.

2. Pessar nach Anspruch 1, dadurch gekennzeichnet, dass die Mitte der kreisförmigen Öffnung (7) im wesentlichen auf der von vorn nach hinten verlaufenden Achse liegt.

3. Pessar nach Anspruch 1 oder 2, dadurch gekennzeichnet, dass die Mitte der kreisförmigen Öffnung (7) der Hinterseite (4) etwas näher liegt, als der Vorderseite (3).

4. Pessar nach einem der vorhergehen den Ansprüche, dadurch gekennzeichnet, dass die kreisförmige Öffnung (7) von einem Ring (8) begrenzt ist, der mittels wenigstens eines Steges (9, 10, 11) mit einem Umfangsrahmen (12) verbunden ist, welchletzterer mit dem oder den Verbindungssteg bzw. -stegen (9, 10, 11) die Durchbrüche (13, 14, 15) für den Ausfluss der Vaginalsekrete ausbildet.

5. Pessar nach Anspruch 4, dadurch gekennzeichnet, dass einer der Verbindungsstege (9) zwischen dem Ring (8) und der Vorderseite (3) angeordnet ist.

6. Pessar nach Anspruch 5, dadurch gekennzeichnet, dass die Ränder des genannten Verbindungssteges (9) vom Ring (8) ausgehend gegen die Vorderseite auseinanderlaufen.

7. Pessar nach Anspruch 5 oder 6, dadurch gekennzeichnet, dass die Ränder des Verbindungssteges (9) auseinanderlaufen, bis sie sich entsprechend mit dem Umfangsrahmen (12) in Höhe des Vorderabschnitts der Querseiten (1, 2) vereinigen.

8. Pessar nach Anspruch 5, 6 oder 7, dadurch gekennzeichnet, dass es darüber hinaus zwei weitere Verbindungsstege (10, 11) enthält, die im wesentlichen zueinander symmetrisch zur von der Vorderseite zur Hinterseite laufenden Achse liegen und jeweils den Ring (8) mit dem Umfangsrahmen (12) in Höhe der Enden der Hinterseite (4) verbinden.

## Claims

1. Pessary usable in particular for the treatment of cervico-isthmic insufficiency during pregnancy, formed by a substantially trapezoidal shaped plate, defined by two lateral sides (1, 2), a concave front side (3) and a rear side (4) also concave but longer than the front side and having, on the one hand, a circular orifice (7) for receiving the cervix of the uterus and, on the other hand, bores (13, 14, 15) for the flow of the vaginal secretions, characterized in that the two lateral sides are of convex shape.

2. Pessary according to claim 1, characterized in that the center of the circular orifice (7) is situated substantially on the front-rear axis.

3. Pessary according to claim 1 or 2, characterized in that the center ot the circular orifice (7) is slightly closer to the rear side (4) than the front side (3).

4. Pessary according to any one of the preceding claims, characterized in that the circular orifice (7) is defined by a ring (8) connected by one branch at least (9, 10, 11) to a peripheral frame (12), this latter defining with the connecting branch or branches (9, 10, 11), the bores (13, 14, 15) for the flow of the vaginal secretions.

5. Pessary according to claim 4, characterized in that one of the connecting branches (9) is situated between the ring (8) and the front side (3).

6. Pessary according to claim 5, characterized in that the edges of said connecting branch (9) diverge from the ring (8) towards the front side (3).

7. Pessary according to claim 5 or 6, characterized in that the edges of the connecting branch (9) diverge until they join respectively the peripheral frame (12) at the level of the front part ot the lateral sides (1, 2).

8. Pessary according to claims 5, 6, or 7, characterized in that it further comprises two other connecting branches (10, 11) disposed substantially symmetrically on each side of the front-rear axis and connecting respectively the ring (8) to the peripheral frame (12) at the level of the ends of the rear side (4).

FIG. 2

FIG. 1

FIG. 3